# EUROPEAN PATENT SPECIFICATION

(11) **EP 4 197 578 B1**
(45) Date of publication and mention of the grant of the patent: **29.01.2025**
(21) Application number: 22207159.9
(22) Date of filing: 14.11.2022
(51) Int. Cl.: A61M 16/04

(54) **ENDOTRACHEAL TUBE**
ENDOTRACHEALTUBUS
TUBE ENDOTRACHEAL

(30) Priority: 14.12.2021 IT 202100031256
(43) Date of publication of application: 21.06.2023
(73) Proprietor: Med-Europe European Medical Supplies S.r.l., 40121 Bologna (IT)
(72) Inventor: CHECCACCI CARBONI, Stefano, 35018 San Martino Di Lupari PD (IT); BONALDI, Efrem, 37047 San Bonifacio VR (IT); GENNARO, Paolo, 35132 Padova (IT)
(74) Representative: Modiano, Micaela Nadia

(56) References cited:
- EP-A1- 0 102 422
- EP-A2- 0 928 620
- WO-A1-2008/110377
- WO-A1-2021/076971
- WO-A1-91/12844
- US-A1- 2005 133 037
- US-A1- 2008 142 003
- US-A1- 2011 061 658
- US-A1- 2015 306 328
- US-A1- 2016 250 433
- US-A1- 2016 287 826
- US-B1- 7 946 981

## Description

The present invention relates to an endotracheal tube.

In the field of medicine, the mechanical (or assisted) ventilation technique is well known, and is used to enable a person to breathe who is partially or completely incapable of doing so on their own, owing to various diseases or critical conditions.

According to the implementation method currently most favored, mechanical ventilation is practiced by connecting the respiratory tract of the patient with a special device (called a mechanical ventilator), which pumps air into the lungs according to operational parameters regulated by a special electronic unit, which are controlled by sensors that detect the condition of the patient instant by instant.

The connection is made using an endotracheal tube, which in the intubation maneuver is inserted into the mouth or nose of the patient, until the distal end reaches the trachea, while at the other end the proximal end is associated with the mechanical ventilator.

Patients subjected to these treatments are typically admitted to dedicated wards of hospitals, called intensive therapy wards, which recently became sadly famous because of the COVID-19 pandemic that exploded in recent years.

The endotracheal tube therefore plays a crucial role in the context described above, but it presents a number of criticalities for which an optimal solution has not yet been found.

In fact, in order to allow an easy introduction into the respiratory tract, the tube must ensure adequate resistance to deformations (in particular to any kinking, which tends to occlude the transverse cross-section), but the solution most commonly adopted in this regard, which consists in the adoption of a material with high rigidity for the tube, presents some serious contraindications.

After introduction, the tube is intended to be left in position for some time (possibly several days) in the respiratory tract of the patient. Not infrequently therefore, the rigidity that distinguishes the tube is the source of stress, trauma and/or lesions, for the mucous membranes and the inner walls of the anatomical cavities involved, which can even lead to sores, rendering another treatment (or an operation) necessary for healing.

The solutions adopted up to the present day to overcome the above-mentioned drawbacks are based on the use of guide pins, introducers and/or probes fitted with video cameras, which facilitate the step of insertion even of deformable tubes, but which have high costs, and are scantly indicated for activities that are carried out increasingly often.

Endotracheal tubes and similar devices (e.g. LMA or tracheostomy tubes) comprising helical reinforcements are disclosed for example in WO2008/110377 A1, WO91/12844 A1, US2005/133037 A1, US 2011/061658 A1, EP0928620 A2, US2008/142003 A1, EP0102422 A1 or WO2015/075412 A1.

The aim of the present invention is to solve the above-mentioned problems, by providing an endotracheal tube that can be introduced into and left in the respiratory tract of a patient easily, without causing lesions or trauma to the patient during insertion into the respiratory tract, and/or for the duration it is subsequently left there.

Within this aim, an object of the invention is to provide an endotracheal tube that makes it possible to easily carry out intubation maneuvers, thus reducing or preventing the danger of kinking the tube.

Another object of the invention is to provide an endotracheal tube that can be left in use for an extended length of time, without being the source of lesions, trauma or sores.

Another object of the invention is to provide an endotracheal tube that is low-cost but with a high reliability of operation.

Another object of the invention is to provide an endotracheal tube that adopts an alternative technical and structural architecture to those of conventional tubes.

Another object of the invention is to provide a tube that can be easily implemented using elements and materials that are readily available on the market.

Another object of the invention is to provide a tube that is certain to be safe in use.

This aim and these and other objects which will become more apparent hereinafter are achieved by an endotracheal tube, comprising at least one main tubular body, configured for the at least partial insertion into the respiratory tract of a patient, through the mouth or the nose, characterized in that it comprises at least one tubular reinforcement skeleton for said body, at least one useful portion of said skeleton being constituted by at least one first filament which is wound in a helical manner around the longitudinal axis of said body and being selectively disengageable from said body, for its removal after the completion of the step of insertion of said body into the respiratory tract of the patient.

The invention is defined in the appended independent claim 1. Preferred embodiments are matter of the dependent claims.

Further characteristics and advantages of the invention will become more apparent from the description of a preferred, but not exclusive, embodiment of the endotracheal tube according to the invention, which is illustrated by way of non-limiting example in the accompanying drawings wherein:
Figure 1 is a side view of the endotracheal tube according to the invention;
Figures 2 to 4 show the use of the endotracheal tube of Figure 1 on a patient, shown in cross-section taken along a sagittal plane;
Figure 5 shows, similarly to Figure 4, the final step of using the endotracheal tube, but provided according to an alternative version to the one in the previous figures.

With reference to the figures, the reference numeral 1 generally designates an endotracheal tube which comprises at least one main tubular body 2, which is configured for the at least partial insertion thereof into the respiratory tract of a patient A, through the mouth B (oral cavity) or the nose C of the patient. In particular, in the preferred application the tube 1 is intended to be used to perform the intubation maneuver and so enable the connection between the respiratory tract and a mechanical ventilator (not shown in the accompanying figures), for the purpose of performing mechanical (or assisted) ventilation on the patient A.

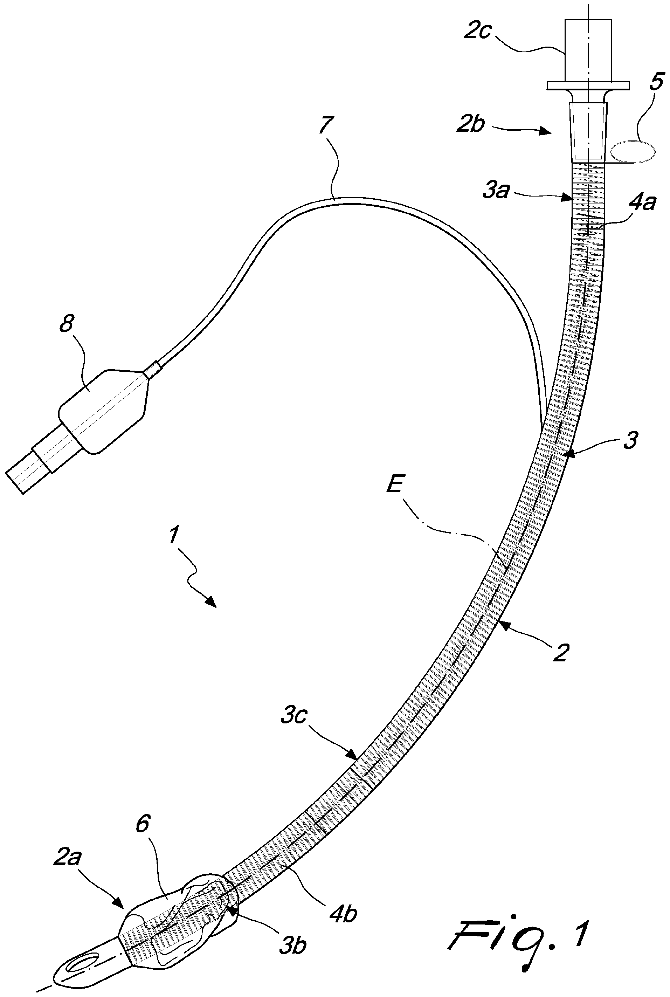

In this context, the insertion into the respiratory tract is stopped when a distal segment 2a of the body 2 reaches the trachea D of the patient A; the length of the body 2 (the dimension measured along the longitudinal axis E of the body 2 proper, shown for the sake of simplicity only in Figure 1) is chosen to be such as to leave in any case at least one proximal segment 2b outside of the mouth B (when the distal segment 2a is in the trachea D) or of the nose C. By "segment" (proximal or distal) what is meant here is an end fraction of the body 2, of lesser dimensions than the overall length of the body 2.

Furthermore, the proximal segment 2b is associated with a connector 2c, which enables the connection to the mechanical ventilator.

It should be noted in any case that the scope of protection also extends to other uses of the tube 1, if the requirements advise it and the practical conditions render it possible. In some cases, the possibility is not ruled out of inserting the body 2 until the proximal segment 2b is in the mouth B or in the nose C (while still keeping it, preferably, accessible from outside, for reasons that will become clear in the following pages).

It should moreover be noted that in the present discussion the terms "proximal" and "distal" are used in their generally accepted meanings in the sector referred to: they therefore refer to those parts of any object or instrument that, at least during use, are located respectively toward the person who holds it ("proximal") or toward the patient A ("distal").

It should likewise be noted that where mention is made of characteristics or aspects of the invention that arise "during use", reference will be made to the condition in which the tube 1 is in fact inserted in the respiratory tract (with intubation completed, for example).

According to the invention, the tube 1 comprises at least one tubular reinforcement skeleton 3 (reinforcement in the sense of radial/transverse stiffening) for the body 2; in other words, the skeleton 3 is configured to increase the transverse (or radial) rigidity of the body 2 and therefore its resistance to kinking stresses, which otherwise could occlude the passage cross-section of the body 2 proper (and in general of the tube 1), thus facilitating the step of insertion of the tube 1 itself into the respiratory tract and in particular reducing or preventing the danger of kinking (as this phenomenon is termed in the sector).

At least one useful portion of the skeleton 3 is constituted by at least one first filament 4a which is wound in a helical manner around the longitudinal axis E of the body 2 and is selectively disengageable from the body 2 proper. In this manner, it is possible to remove the first filament 4a and in general at least the useful portion after the completion of the step of insertion of the body 2 into the respiratory tract of the patient A.

In fact, the properties just illustrated, which are in fact conferred by the skeleton 3, are required in particular (or only) during the step of insertion: subsequently the body 2 can be left for a lengthy period of time (even several days) in the placement obtained, and the resistance to kinking could become a problem (at least for part of the tube 1), in that the body 2 thus reinforced could bring considerable pressures to bear on the mucous membranes and anatomical tissues of the patient A.

The ability to disengage and therefore remove one or more useful portions of the skeleton 3 (or the entire skeleton 3) from the tube 1 reduces or cancels out this risk, effectively achieving the set aim from this point onward. As a consequence of the removal in fact, the properties of the tube 1 are (only) those conferred by the body 2 (at least in the fraction thereof with no skeleton 3), which are therefore chosen so as not to be capable of representing a danger to the mucous membranes and anatomical tissues of the patient A.

As just mentioned, it is emphasized that there can be two or more useful, and therefore removable, portions (two or more longitudinal fractions of the skeleton 3, possibly separated by respective fractions that are instead intended to remain in the respiratory tract during use).

The helical shape or arrangement of the first filament 4a ensures that the increased resistance to kinking is a property that is uniformly distributed along the entire length of the tube 1 (for as long as the useful portion is kept engaged with the body 2) and at the same time facilitates disengagement.

It should be noted from this point onward that it is envisaged to make the body 2 from polymeric material, and preferably it is made of a material selected from the group comprising polyvinyl chloride (PVC) and polyurethane (PU or PUR), with a Shore hardness for example equal to 40-60 A.

By contrast, at least the first filament 4a (or the entire skeleton 3) is made of metal, and preferably is made of stainless steel (with superior rigidity and hardness).

The possibility is not ruled out however of using materials of another type, on condition however that the skeleton 3 can effectively contrast the kinking of the body 2 (the occlusion of its transverse cross-section) and that the latter does not generate trauma, lesions or in general unwanted stresses on the anatomical tissues and mucous membranes of the patient A.

In more detail, it should be noted that the resistance conferred by the skeleton 3 can be more or less high, according to the specific requirements: it will need to be sufficient to facilitate the insertion maneuver (while preventing the tube 2 from kinking during the insertion), but not so high as to compromise the insertion or to make it necessary to use an introducer.

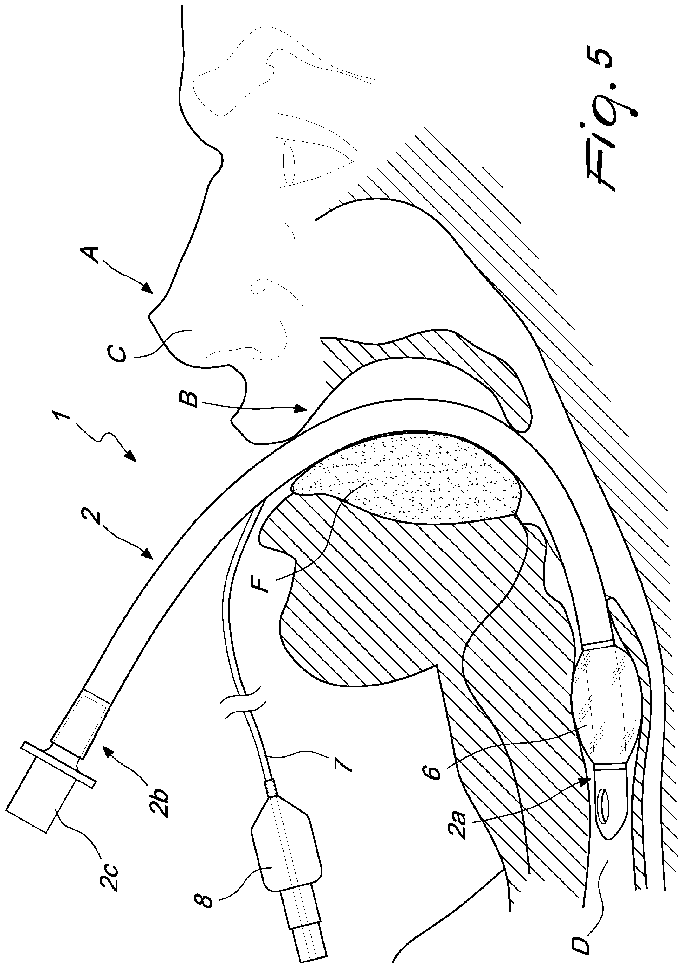

In a first embodiment of the invention (corresponding to Figure 5), at least the first filament 4a extends substantially for the entire longitudinal extension of the body 2, and therefore the skeleton 3 is in this case fully disengageable and removable from the body 2 (and from the tube 1), in order to therefore leave solely the body 2 itself in the respiratory tract of the patient A, after completion of the step of introduction.

In a second embodiment, the skeleton 3 is constituted substantially by a proximal part 3a (which contains the proximal end of the skeleton 3) and a distal part 3b (which contains the distal end of the skeleton 3), which are arranged in sequence along the longitudinal extension of the body 2 and are mutually disengaged (at respective adjacent ends which define a decoupling point for the skeleton 3). In such case, the useful portion is preferably constituted by the distal part 3b, while the proximal part 3a is (preferably but not necessarily) made integral with the body 2 (and is intended to be left inside the respiratory tract of the patient A for the entire duration of use of the tube 1). The length of the proximal part 3a and of the distal part 3b, and therefore the position in the respiratory tract where the decoupling point is located, can be any: preferably however, the length of the proximal part 3a is chosen such as to ensure that during use it extends along the entire length of the tongue F (and not beyond). In fact, the corresponding fraction of the body 2 is the fraction that during use is intended to be subjected to the greatest deformation (or which in any case is the fraction that is bent the most): the choice not to remove the proximal part 3a of the skeleton 3 prevents the risk that buckling could arise also during use (which could give rise to choking or kinking phenomena) along the corresponding fraction of the body 2.

In a third embodiment, illustrated in the accompanying Figures 1-4, which in any case does not exhaust the possibilities covered in the scope of protection claimed herein, the skeleton 3 comprises at least one proximal part 3a, an intermediate part 3c and a distal part 3b, which are arranged in sequence along the longitudinal extension of the body 2 and are mutually disengaged. Effectively, with respect to the previous embodiment, the skeleton 3 has at least one additional section 3c, intermediate, which is interposed between the other two. In this case, the useful portion is preferably constituted by the intermediate part 3c, while the proximal part 3a and the distal part 3b are (preferably but not necessarily) made integral with the body 2 (and are intended to be left inside the respiratory tract of the patient A for the entire duration of use of the tube 1).

Again, the parts 3a, 3c, 3b are disengaged in pairs at respective adjacent ends (which define corresponding decoupling points for the skeleton 3), and the length of the three parts 3a, 3c, 3b can be any.

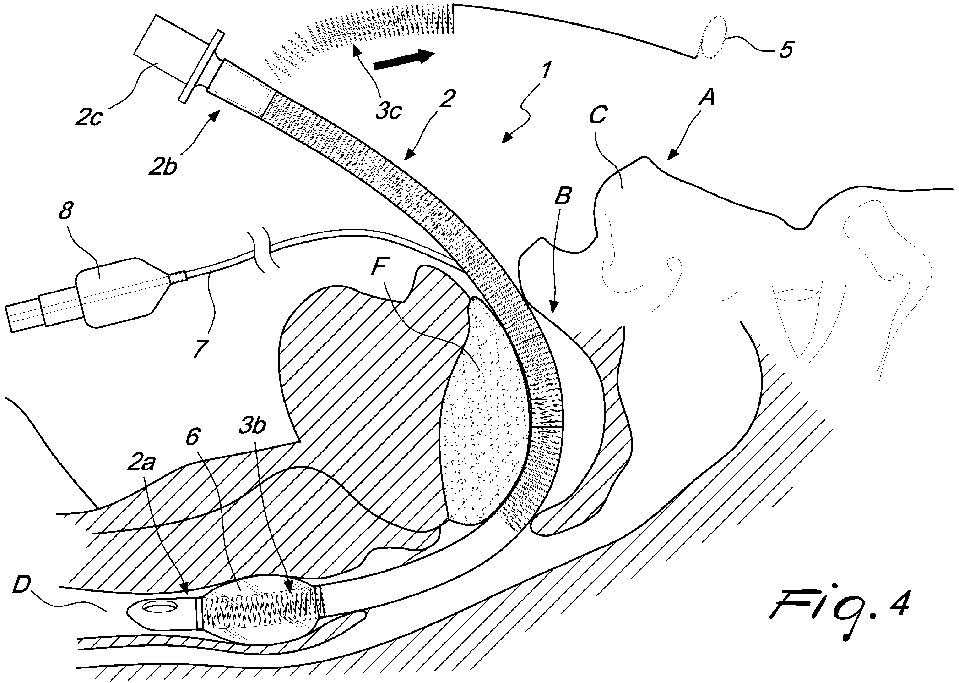

At the same time, in the preferred embodiment the length of the proximal part 3a is chosen according to the criterion illustrated for the previous embodiment (as incidentally can be clearly seen in Figure 4), and we will return later to the preferred length of the distal part 3b (and the reasons why it can be preferred to leave it in the respiratory tract even during use).

In general however, it should be noted that in the present discussion the wordings "proximal part 3a" and "distal part 3b" indicate any desired fractions of length of the skeleton 3, which in any case comprise the respective ends (proximal and distal): as has been seen, it is possible for the proximal part 3a and the distal part 3b to represent the entire longitudinal or axial extension of the skeleton 3, or for there to be an intermediate part 3c interposed between them.

It should likewise be noted that, in other contexts, it may instead be preferable to remove the proximal part 3a of the skeleton 3 (even only in the region of the teeth), for example if it is thought that the patient might bite, because in such a case throttling could arise in any case. In such case, the distal part 3b only of the skeleton 3 can for example be left in the respiratory tract during use.

More generally, the possibility is not ruled out of another useful portion being designed to be removed, different from the possibilities listed above, or even, as already mentioned, that there are two or more useful portions that can be removed. Any choice in this regard should be understood to be comprised in the scope of protection claimed herein.

The length of the useful portion (or of each useful portion), that can be removed, can be chosen as a function of the hardness of the material used.

In turn, typically the skeleton 3 can have a length that essentially corresponds to that of the body 2 (or at least to the fraction of the latter that is intended to be left in the respiratory tract during use), although different practical solutions are not ruled out.

In a first possible practical placement, according to the invention, the skeleton 3 is accommodated (contained) in the thickness of the body 2 (between the internal lateral surface and the external lateral surface): in such case, the first filament 4a is longitudinally extractable from the thickness (in order to selectively obtain the removal thereof). Effectively, in such solution the removal consists in, by exerting a pull from outside on at least one limb thereof, the first filament 4a being peeled off (preferably with a helical motion) from the body 2, typically leaving in the latter (once removal is complete) a form of groove (helical), embedded in the thickness, left by the previous placement of the first filament 4a.

In a second possible practical placement (which however is not covered by the scope of protection claimed herein), the skeleton 3 is engaged with either the internal lateral surface or the external lateral surface of the body 2. For example, the skeleton 3 can be applied on the chosen lateral surface using an adhesive or in any case an adhesive film. Obviously, the engagement between at least the useful portion and the chosen surface will be of the removable type.

The removal of the first filament 4a is in any case obtained by exerting a pull from outside on at least one limb of the first filament 4a, so as to cause the release thereof from the body 2.

Conveniently, in an embodiment of significant practical interest, illustrated in the accompanying Figures 1-4 for the purposes of non-limiting example of application of the invention, at least one second portion of the skeleton 3, which is separate from the useful portion and is integral with the body 2, comprises at least one second filament 4b which is wound in a helical manner around the longitudinal axis E of the body 2. Effectively, the second portion can coincide with the proximal part 3a and/or with the distal part 3b (in the solution of Figure 4) and in this case has a shape structure similar to that of the useful portion: the skeleton 3 will therefore be constituted by a plurality of filaments 4a, 4b (which are made preferably of the same material, are arranged in series and are contiguous, although separate).

In a different embodiment, at least one second portion of the skeleton 3, which is separate from the useful portion and is integral with the body 2, is substantially constituted by a mesh composed of a plurality of wires that are mutually interwoven and wound around the longitudinal axis E of the body 2.

It should in any case be noted that the second portion or portions can be provided in other ways as well, other than the two options described above, while remaining within the scope of protection claimed herein.

Precisely with reference to the scope of protection that the present discussion sets out to claim, such scope is understood to include tubes 1 that have any number of filaments 4a, 4b: in particular, the possibility exists that the useful portion of the skeleton 3 comprises one or more first filaments 4a and that each second portion comprises one or more second filaments 4b (in the embodiment that makes use of these latter items, obviously). Any reference that has been made or will be made in the present discussion to "a" filament 4a, 4b should therefore be understood to being extended to all (or some of) the filaments 4a, 4b that may be envisaged.

Usefully, in order to facilitate the removal at least of the useful portion, the tube 1 comprises a grip element 5, which is kept protruding from a proximal segment 2b of the body 2 and is anchored to the first filament 4a (for example by virtue of an additional connecting wire accommodated in the thickness of the wall of the body 2). The element 5 can be gripped by an operator (typically, but not necessarily, an anesthetist or a medical expert) in order to remove at least the first filament 4a (it is sufficient to exert a pulling action on the element 5 to cause the extraction or in any case the release from the body 2 and therefore the removal).

The shape of the element 5 can be any, depending on the specific requirements: in the solution of the accompanying figures it is ring-shaped, easily grippable and at the same time free from sharp points which would be potentially dangerous. However, the possibility is not ruled out of giving the element 5 a different shape. It is envisaged that the first filament 4a, the element 5 and the connecting wire will be made of the same material and/or they will be a sort of single component, just as the possibility is not ruled out of making them of different materials, but still rigidly coupled.

Conveniently, the tube 1 comprises an inflatable enclosure 6 (a form of balloon), which externally surrounds the distal segment 2a of the body 2 and which is associated with an inflation line 7 (which is also, for example, at least partially embedded in the thickness of the body 2), for stabilizing the body 2 in the respiratory tract (typically, in the trachea D) of the patient A. The stabilization is obtained by inflating the enclosure 6, until it is brought into contact with the mucous membranes. In order to facilitate the inflation step, there will be a sort of inflatable lung 8 mounted on the other end of the line 7, and provided with unidirectional valves arranged appropriately, in order to be able to fill it with air to be sent progressively to the enclosure 6, by applying pressure to the lung 8, which behaves like a kind of pump.

As the accompanying Figure 4 shows, the distal part 3b (in the embodiment in which it is not removed during use) has a length that corresponds to the fraction of body 2 that is affected by the enclosure 6: in fact, such fraction of body 2 does not press in any way against the anatomical walls (given the presence of the enclosure 6 that surrounds it) and therefore the mechanical properties conferred by the distal part 3b do not cause discomfort.

The use of the tube according to the invention is effectively already clear from the foregoing description, but below a brief summary is given in any case.

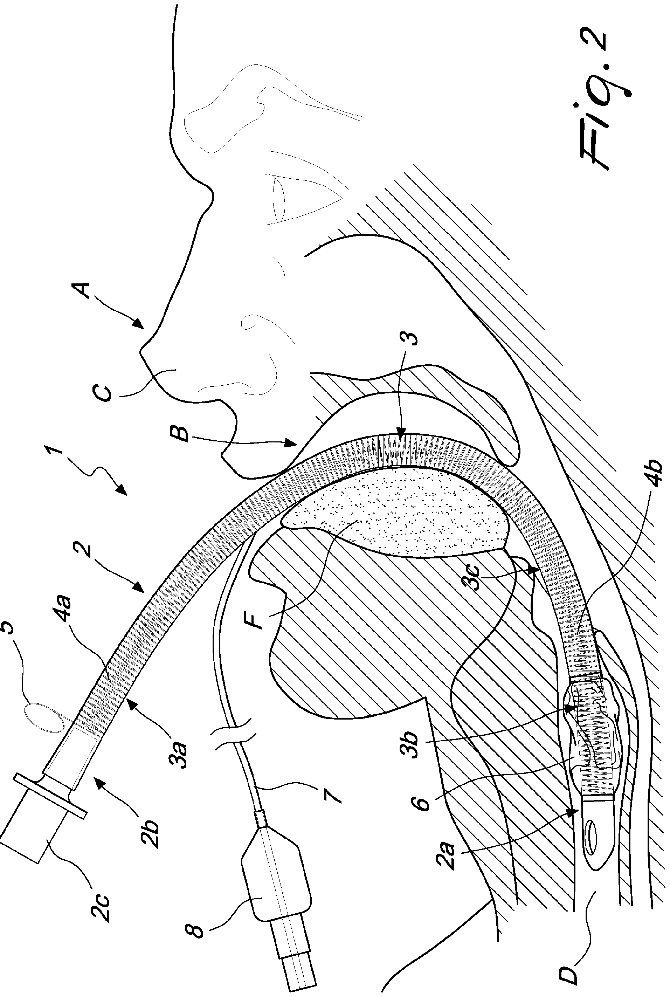

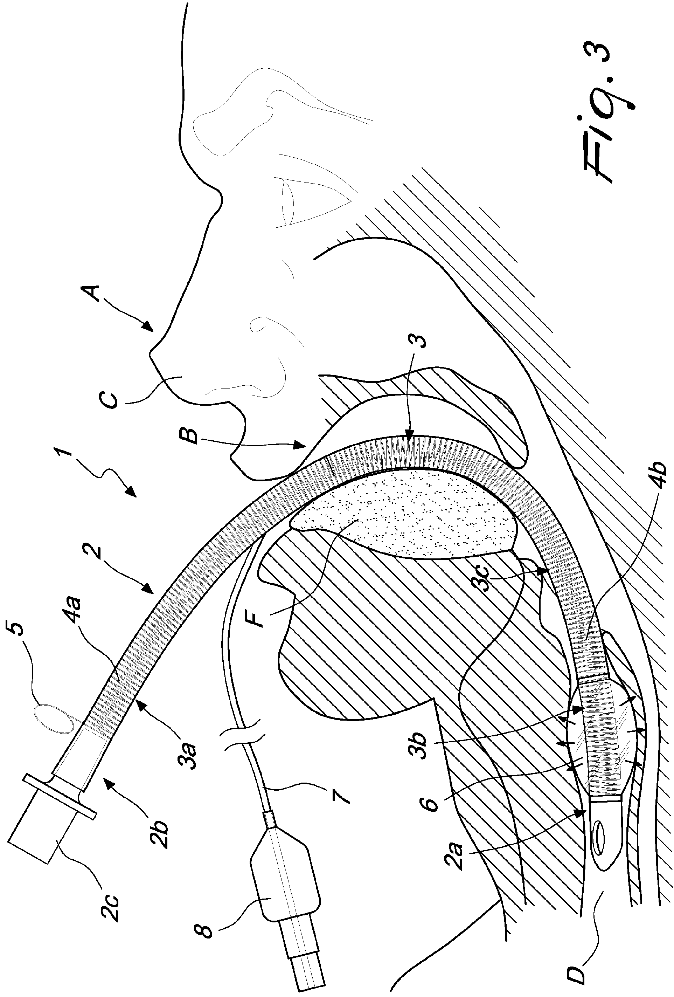

The tube 1 can be inserted into the respiratory tract of a patient A, gaining access thereto through the mouth B (as in the accompanying figures) or the nose C, and until the distal segment 2a is brought into the trachea D (Figure 2). Once insertion is complete, preferably the tube 1 is stabilized (and the distal segment 2a in particular), for example by inflating the enclosure 6 (Figure 3), in anticipation of maintaining the tube 1 in such placement for a more or less extended period.

The body 2 (tube and so on) is effectively responsible for the connection of the respiratory tract with the outside: in this regard, the body 2 is connected to a mechanical ventilator, at a connector 2c.

During the step of insertion, the entire skeleton 3 is kept associated with the body 2, conferring greater resistance to kinking (greater transverse/radial rigidity) on the tube 1 than that offered by the body 2 alone, and this in fact simplifies the step of introduction, which otherwise could be complicated owing to bending or unwanted kinking phenomena.

In a very specific manner, once insertion is complete the invention offers the possibility of disengaging from the body 2 and partially or fully removing the skeleton 3 (at least the useful portion(s) thereof), simply by peeling off, or in any case extracting, the first filament 4a. The grip element 5 facilitates this activity, by offering the operator (be they an anesthetist, a medical expert, or other kind of operator) an even easier gripping point. Thus, only the softer body 2 (Figure 5) is left inside the respiratory tract, with at most only the fractions of the skeleton 3 that have been chosen to be made non-removable (for example, only the proximal part 3a and/or only the distal part 3b).

Thus, for the entire length of time for which it is intended to leave the tube 1 in the respiratory tract, the risk of undergoing trauma or stress caused by an excessive rigidity of the tube 1 is canceled out or reduced, in that during use the latter is made up solely of the body 2, which is soft and flexible (at the most, with the remaining fractions of skeleton 3 left inside the respiratory tract, for the reasons already explained).

At the end of the use, the tube 1 can be simply removed by pulling the body 2 out from the respiratory tract (obviously not before deflating the enclosure 6 or in any case deactivating the chosen stabilization system).

In practice it has been found that the endotracheal tube according to the invention fully achieves the set aim, in that the use of a skeleton 3 to reinforce the body 2, which comprises at least one useful portion that is selectively disengageable and therefore removable, makes it possible to provide an endotracheal tube 1 that can be first introduced into and then left inside the respiratory tract of a patient A easily (by virtue of the skeleton 3), but without causing lesions or trauma to the latter (by virtue of the removal at least of the useful portion) during insertion into the respiratory tract, and/or for the duration it is subsequently left there.

In particular, the particularities just described make it possible, in the preferred application, to easily carry out an intubation maneuver, in that the skeleton 3 facilitates the introduction of the tube 1 by enabling the (optimal) execution of the maneuver even for any medical anesthetist or personnel with sufficient qualifications, thus reducing or preventing the danger of kinking.

The removal of the skeleton 3 (at least of its useful portion) makes it possible during use to leave the tube 1 (in the respiratory tract) for an extended length of time, without being a source of lesions, trauma or sores.

The simplicity of the invention is first and foremost a guarantee of reliability in operation and at the same time of economy of the solution; in particular it is emphasized that an optimal introduction can be obtained without resorting to spindles or video cameras (which in conventional solutions, by contrast, raise the price appreciably), but only with the skeleton 3, which is then at least partially removed.

The invention thus conceived is susceptible of numerous modifications and variations, all of which are within the scope of the appended claims.

In the embodiments illustrated, individual characteristics shown in relation to specific examples may in reality be substituted with other, different characteristics, existing in other embodiments.

In practice, the materials employed, as well as the dimensions, may be any according to requirements and to the state of the art.

This application claims priority from Italian Patent Application No. 102021000031256.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

The invention is defined by the claims which follow.

## Claims

1. An endotracheal tube, comprising at least one main tubular body (2), configured for the at least partial insertion into the respiratory tract of a patient (A), through the mouth (B) or the nose (C), said endotracheal tube (1) comprising at least one tubular reinforcement skeleton (3) for said body (2), at least one useful portion of said skeleton (3) being constituted by at least one first filament (4a) which is wound in a helical manner around the longitudinal axis (E) of said body (2) and being selectively disengageable from said body (2), for its removal after the completion of the step of insertion of said body (2) into the respiratory tract of the patient (A), **characterized in that** said skeleton (3) is accommodated within the thickness of said body (2), said at least one first filament (4a) being longitudinally extractable from said thickness.

2. The tube according to claim 1, **characterized in that** said at least one first filament (4a) extends substantially over the entire longitudinal extension of said body (2), said skeleton (3) being entirely disengageable from said body (2).

3. The tube according to claim 1, **characterized in that** said skeleton (3) is constituted substantially by a proximal part (3a) and by a distal part (3b), which are arranged in sequence along the longitudinal extension of said body (2) and are mutually disengaged, said useful portion being constituted by said distal part (3b).

4. The tube according to claim 1, **characterized in that** said skeleton (3) comprises at least one proximal part (3a), an intermediate part (3c), and a distal part (3b), which are arranged in sequence along the longitudinal extension of said body (2) and are mutually disengaged, said useful portion being constituted by said intermediate part (3c).

5. The tube according to one or more of claims 3-4, **characterized in that** at least one second portion of said skeleton (3), which is separate from said useful portion and is integral with said body (2), comprises at least one second filament (4b) which is wound in a helical manner around the longitudinal axis (E) of said body (2).

6. The tube according to one or more of claims 3-4, **characterized in that** at least one second portion of said skeleton (3), which is separate from said useful portion and is integral with said body (2), is substantially constituted by a mesh composed of a plurality of wires that are mutually interwoven and wound around the longitudinal axis (E) of said body (2).

7. The tube according to one or more of the preceding claims, **characterized in that** it comprises a grip element (5), which is kept protruding from a proximal segment (2b) of said body (2) and is anchored to said at least one first filament (4a), said element (5) being grippable by an operator in order to remove said at least one first filament (4a).

8. The tube according to one or more of the preceding claims, **characterized in that** it comprises an inflatable enclosure (6), which is wrapped externally around a distal segment (2a) of said body (2) and is associated with an inflation line (7), for the stabilization of said body (2) in the respiratory tract of the patient (A).

## Patentansprüche

1. Ein Endotrachealtubus, der mindestens einen schlauchförmigen Hauptkörper (2) umfasst, ausgebildet zum zumindest partiellen Einführen in die Atemwege eines Patienten (A) durch den Mund (B) oder die Nase (C); wobei der Endotrachealtubus (1) mindestens ein schlauchförmiges Verstärkungsgerüst (3) für den Körper (2) umfasst, wobei mindestens ein nützlicher Abschnitt des Gerüsts (3) aus mindestens einem ersten Draht (4a) besteht, der spiralförmig um die Längsachse (E) des Körpers (2) gewunden wird und wahlweise von dem Körper (2) getrennt werden kann zum Entfernen nach Beendigung des Schritts des Einführens des Körpers (2) in die Atemwege des Patienten (A); **dadurch gekennzeichnet, dass** das Gerüst (3) in der Dicke des Körpers (2) untergebracht ist, wobei der mindestens eine erste Draht (4a) in Längsrichtung aus der Dicke herausziehbar ist.

2. Der Tubus gemäß Anspruch 1, **dadurch gekennzeichnet, dass** der mindestens eine erste Draht (4a) sich im Wesentlichen über die gesamte Längsausdehnung des Körpers (2) erstreckt, wobei das Gerüst (3) vollständig von dem Körper (2) trennbar ist.

3. Der Tubus gemäß Anspruch 1, **dadurch gekennzeichnet, dass** das Gerüst (3) im Wesentlichen aus einem proximalen Teil (3a) und einem distalen Teil (3b) besteht, die entlang der Längsausdehnung des Körpers (2) hintereinander angeordnet und voneinander getrennt sind, wobei der nützliche Abschnitt in dem distalen Teil (3b) besteht.

4. Der Tubus gemäß Anspruch 1, **dadurch gekennzeichnet, dass** das Gerüst (3) mindestens einen proximalen Teil (3a), einen mittleren Teil (3c) und einen distalen Teil (3b) umfasst, die entlang der Längsausdehnung des Körpers (2) hintereinander angeordnet und voneinander getrennt sind, wobei der nützliche Abschnitt in dem mittleren Teil (3c) besteht.

5. Der Tubus gemäß einem oder mehreren der Ansprüche 3-4, **dadurch gekennzeichnet, dass** mindestens ein zweiter Abschnitt des Gerüsts (3), der von dem nützlichen Abschnitt separat und mit dem Körper (2) integral ist, mindestens einen zweiten Draht (4b) umfasst, der spiralförmig um die Längsachse (E) des Körpers (2) gewunden ist.

6. Der Tubus gemäß einem oder mehreren der Ansprüche 3-4, **dadurch gekennzeichnet, dass** mindestens ein zweiter Abschnitt des Gerüsts (3), der von dem nützlichen Abschnitt separat und mit dem Körper (2) integral ist, im Wesentlichen aus einem Gitter besteht, das aus einer Vielzahl von Drähten besteht, welche miteinander verwoben und um die Längsachse (E) des Körpers (2) gewunden sind.

7. Der Tubus gemäß einem oder mehreren der obigen Ansprüche, **dadurch gekennzeichnet, dass** er ein Griffelement (5) umfasst, das von einem proximalen Segment (2b) des Körpers (2) vorstehend gehalten wird und an dem mindestens einen ersten Draht (4a) verankert ist; wobei das Element (5) von einem Bediener ergriffen werden kann, um den mindestens einen ersten Draht (4a) zu entfernen.

8. Der Tubus gemäß einem oder mehreren der obigen Ansprüche, **dadurch gekennzeichnet, dass** er eine aufblasbare Umhüllung (6) umfasst, welche außen um ein distales Segment (2a) des Körpers (2) gewickelt und zur Stabilisierung des Körpers (2) in den Atemwegen des Patienten (A) mit einem Füllschlauch (7) verbunden ist.

## Revendications

1. Tube endotrachéal comprenant au moins un corps tubulaire principal (2), configuré pour l'insertion au moins partielle dans les voies respiratoires d'un patient (A), par la bouche (B) ou le nez (C), ledit tube endotrachéal (1) comprenant au moins une structure de renforcement tubulaire (3) pour ledit corps (2), au moins une partie utile de ladite structure (3) étant constituée d'au moins un premier filament (4a) qui est enroulé de manière hélicoïdale autour de l'axe longitudinal (E) dudit corps (2) et pouvant être dégagée de façon sélective dudit corps (2), pour son retrait après l'achèvement de l'étape d'insertion dudit corps (2) dans les voies respiratoires du patient (A), **caractérisé en ce que** ladite structure (3) est logée dans l'épaisseur dudit corps (2), ledit au moins un premier filament (4a) étant extractible de ladite épaisseur de façon longitudinale.

2. Tube selon la revendication 1, **caractérisé en ce que** ledit au moins un premier filament (4a) s'étend substantiellement sur toute l'extension longitudinale dudit corps (2), ladite structure (3) pouvant être dégagée entièrement dudit corps (2).

3. Tube selon la revendication 1, **caractérisé en ce que** ladite structure (3) est constituée substantiellement d'une partie proximale (3a) et d'une partie distale (3b), qui sont disposées en séquence le long de l'extension longitudinale dudit corps (2) et qui sont mutuellement dégagées, ladite partie utile étant constituée par ladite partie distale (3b).

4. Tube selon la revendication 1, **caractérisé en ce que** ladite structure (3) comprend au moins une partie proximale (3a), une partie intermédiaire (3c) et une partie distale (3b), qui sont disposées en séquence le long de l'extension longitudinale dudit corps (2) et qui sont mutuellement dégagées, ladite partie utile étant constituée par ladite partie intermédiaire (3c).

5. Tube selon l'une ou plusieurs des revendications 3 à 4, **caractérisé en ce qu'**au moins une deuxième partie de ladite structure (3), qui est distincte de ladite partie utile et est intégrée audit corps (2), comprend au moins un deuxième filament (4b) qui est enroulé de manière hélicoïdale autour de l'axe longitudinal (E) dudit corps (2)

6. Tube selon l'une ou plusieurs des revendications 3 à 4, **caractérisé en ce qu'**au moins une deuxième partie de ladite structure (3), qui est distincte de ladite partie utile et est intégrée audit corps (2), est constituée substantiellement d'un treillis composé d'une pluralité de fils métalliques qui sont mutuellement entrelacés et enroulés autour de l'axe longitudinal (E) dudit corps (2).

7. Tube selon l'une ou plusieurs des revendications précédentes, **caractérisé en ce qu'**il comprend un élément de préhension (5), qui est maintenu saillant depuis un segment proximal (2b) dudit corps (2) et est ancré dans ledit au moins un premier filament (4a), ledit élément (5) pouvant être saisi par un opérateur afin de retirer ledit au moins un premier filament (4a).

8. Tube selon l'une ou plusieurs des revendications précédentes, **caractérisé en ce qu'**il comprend une enveloppe gonflable (6), qui enveloppe extérieurement un segment distal (2a) dudit corps (2) et est associée à une conduite de gonflage (7), pour la stabilisation dudit corps (2) dans les voies respiratoires du patient (A).
